# EUROPEAN PATENT APPLICATION

(11) **EP 4 424 279 A2**
(43) Date of publication of application: **04.09.2024**
(21) Application number: 24169100.5
(22) Date of filing: 08.04.2021
(51) Int. Cl.: A61F 2/24

(54) **STEERABLE DELIVERY APPARATUS FOR AN IMPLANTABLE MEDICAL DEVICE**

(30) Priority: 09.04.2020 US 202063007470 P
(62) Divisional of application: 21722670.3
(71) Applicant: Edwards Lifesciences Corporation, Irvine, CA 92614 (US)
(72) Inventor: Murad, Michael C., IRVINE, CA, 92614 (US)
(74) Representative: Eisenführ Speiser

(57) **Abstract**

A steerable delivery apparatus for an implantable medical device comprises a shaft extending distally from the handle, the shaft having a steering-member lumen extending through a length of the shaft, and a steering member extending through the steering-member lumen, the steering member having a proximal end portion and a distal end portion. A pull ring is disposed along a distal end portion of the shaft, the pull ring comprising a proximal segment having a first diameter and a distal segment having a second diameter smaller than the first diameter. The distal end portion of the steering member is fixed to the distal segment of the pull ring, and an adjustment mechanism is further provided in the handle, the mechanism operatively coupled to the proximal end portion of the steering member and configured to adjust tension in the steering member so as to adjust a curvature of the shaft.

## Description

### CROSS-REFERENCE TO RELATED APPLICATION

The present application claims the benefit of U.S. Provisional Application No. 63/007,470, filed April 9, 2020, which is incorporated herein by reference.

### FIELD

The present disclosure concerns embodiments of a pull ring assembly for a steerable delivery apparatus used for implantation of a medical device, such as a prosthetic heart valve.

### BACKGROUND

Endovascular delivery devices (e.g., catheters) are used in various procedures to deliver prosthetic medical devices or instruments to locations inside the body that are not readily accessible by surgery or where access without surgery is desirable. Access to a target location inside the body can be achieved by inserting and guiding the delivery catheter through a pathway or lumen in the body, including, but not limited to, a blood vessel, an esophagus, a trachea, any portion of the gastrointestinal tract, a lymphatic vessel, to name a few.

In one specific example, a prosthetic heart valve is mounted in a crimped state on the distal end of the delivery device and advanced through the patient's vasculature (e.g., through the femoral artery and the aorta) until the prosthetic valve reaches the implantation site in the heart. The prosthetic valve is then expanded to its functional size, such as by inflating a balloon on which the prosthetic valve is mounted, or by deploying the prosthetic valve from a sheath that extends over the valve so that the prosthetic valve can self-expand to its functional size.

The usefulness of such delivery devices can be limited by their ability to successfully navigate through small vessels and around tight bends in the vasculature, such as around the aortic arch. Accordingly, various techniques have been developed to adjust the curvature of a distal section of the delivery device. One known technique employs a pull ring assembly comprising a pull wire and a pull ring. A distal end of the pull wire is fixedly secured to a steerable section at a distal end of the delivery device via the pull ring and a proximal end of the pull wire is operatively connected to a rotatable adjustment knob on the handle of the delivery device. Rotation of the adjustment mechanism applies a pulling force on the pull wire, which in turn causes the steerable section to bend.

A known method in the art used to manufacture steerable catheters is to form the catheter on a mandrel using three main layers: an inner polymeric layer, a metal braided layer and an outer polyermic layer. The inner polymeric layer is pulled over the mandrel and tightened down. The pull wire is laid axially along the inner layer, often within a groove present on the surface of the mandrel. A polymeric tie layer can be pulled over the inner layer and the pull wire. The braided layer is pulled or braided over the tie layer or directly over the inner layer and the pull wire. A pull ring is slid over the distal end portion of the braided layer and affixed (such as by welding) to a distal end portion of the pull wire. In order to position the distal end portion of the pull wire against the pull ring, an opening in the braided layer can be formed by cutting one or more filaments of the braid and pulling the distal end portion of the pull wire through the opening in the braided layer such that the pull wire extends from the groove of the mandrel through the braided layer and alongside the pull ring. After the braided layer is tightened down, the entire catheter is encased in an outer polymeric layer (typically a thermoplastic material). The outer layer is then encased in heat shrink material and heated. The heat causes the outer layer to flow, which in conjunction with the pressure from the heat shrink material causes the thermoplastic outer layer to flow into the braided layer, forming a shaft having a laminate construction.

Unfortunately, there are several potential drawbacks associated with this technique. For example, the inner diameter of the pull ring must be larger than the outer diameter of the braided layer in order to slide the pull ring over the braided layer. This produces a relatively long transition section of the pull wire where it extends radially inwardly from the pull ring into the groove of the mandrel. This relatively long transition section coupled with the stiffness of the pull wire can result in undesirable canting of the pull ring which can form leak paths through the shaft.

Moreover, the filaments of the braided layer can unravel at the location of the cut, particularly under tensile stress, creating a weak spot at the end of the shaft. In some cases, this can compromise the shaft's ability to hold pressure and in rare circumstances can lead to unpredictable failure loads of the delivery device. Also, the metal ends of the filaments at the location of the cut can be pushed into the inner polymeric layer of the shaft during reflow, creating additional leak paths.

Accordingly, there remains a need for new and improved steerable catheters and methods for their construction.

### SUMMARY

Disclosed herein are embodiments of an improved delivery apparatus for an implantable medical device (e.g., a prosthetic heart valve), as well as related methods for use of such an apparatus in delivering (ore retrieving) an implantable medical device in a patient's body. The delivery apparatus comprises a pull ring assembly comprising a pull ring and a steering member attached to the pull ring. In particular embodiments, the pull ring has a proximal region having a diameter that is large enough to extend over a braided layer of a shaft of the delivery apparatus. The pull ring has a distal region that is smaller in diameter than the proximal region, and an opening where the proximal region transitions to the distal region. The steering member can extend through the braided layer, the proximal region of the pull ring, through the opening of the pull ring and alongside an outer surface of the distal region of the pull ring.

In one representative embodiment, a delivery apparatus for an implantable medical device comprises a handle; a shaft extending distally from the handle, the shaft having a steering-member lumen extending a length of the shaft; a steering member extending through the steering-member lumen, the steering member having a proximal end portion and a distal end portion; and a pull ring disposed along a distal end portion of the shaft, the pull ring comprising a proximal segment having a first diameter and a distal segment having a second diameter smaller than the first diameter. The distal end portion of the steering member is fixed to the distal segment of the pull ring. Further, the handle comprises an adjustment mechanism operatively coupled to the proximal end portion of the steering member and configured to adjust tension in the steering member so as to adjust a curvature of the shaft.

In another representative embodiment, a steerable delivery apparatus for an implantable medical device comprises a handle; a shaft extending distally from the handle, the shaft having a steering-member lumen extending between a distal end portion and a proximal end portion of the shaft, and a braided layer radially outward of the steering-member lumen; a steering member extending through the steering-member lumen, the steering member having a proximal end portion and a distal end portion; and a pull ring coupled to the distal end portion of the shaft. Further, the pull ring comprises a proximal segment having a first diameter and a distal segment having a second diameter smaller than the first diameter, wherein the distal end portion of the steering member is fixed to the distal segment of the pull ring, and wherein a distal end portion of the braided layer extends into the proximal segment of the pull ring and terminates at a location proximal to the distal segment of the pull ring. Furthermore, the handle comprises an adjustment mechanism operatively coupled to the proximal end portion of the steering member, wherein actuation of the adjustment mechanism varies tension in the steering member to vary a curvature of the shaft.

In another representative embodiment, a steerable delivery apparatus for an implantable medical device comprises a handle; a shaft extending distally from the handle, the shaft having a steering-member lumen extending between a distal end portion and a proximal end portion of the shaft, a first layer, a second layer disposed co-axially over the first layer, and a third layer disposed co-axially over the second layer; a steering member extending through the steering-member lumen, the steering member having a proximal end portion and a distal end portion; and a pull ring coupled to the distal end portion of the shaft, the pull ring comprising a proximal segment having a first diameter and a distal segment having a second diameter smaller than the first diameter, wherein the distal end portion of the steering member is fixed to the distal segment of the pull ring, and wherein a distal end portion of the second layer of the shaft extends into the proximal segment of the pull ring and terminates at a location proximal to the distal segment of the pull ring. Further, the handle comprises an adjustment mechanism operatively coupled to the proximal end portion of the steering member, wherein actuation of the adjustment mechanism varies tension in the steering member to vary a curvature of the shaft.

In another representative embodiment, a method of using a catheter device comprises inserting a shaft of the catheter device into the body of a patient, the shaft comprising a steering-member lumen extending there-through, a steering member extending through the steering-member lumen, the steering member having a proximal end portion and a distal end portion, the distal end portion of the steering member fixed to a distal end portion of the shaft via a pull ring, the pull ring comprising a proximal segment having a first diameter and a distal segment having a second diameter, wherein the first diameter is larger than the second diameter; and steering the shaft inside the body of the patient by actuating an adjustment mechanism on a handle of the device outside the body of the patient wherein the adjustment mechanism is operatively coupled to the proximal end portion of the steering member and the actuating includes adjusting tension in the steering member to adjust a curvature of the shaft.

In another representative embodiment, a method for making a steerable catheter apparatus comprises placing a first polymeric layer on a mandrel; placing a steering member longitudinally along the first polymeric layer; placing a braided layer over the first polymeric layer and the steering member; positioning a pull ring relative to the first polymeric layer, the steering member and the braided layer such that a distal end portion of the braided layer extends into a proximal segment of the pull ring, a distal end portion of the steering member extends through the proximal segment of the pull ring, distally beyond the braided layer and alongside a distal segment of the pull ring, wherein proximal segment of the pull ring has a larger diameter than a diameter of the distal segment of the pull ring; placing a second polymeric layer over the braided layer; and bonding the first and second polymeric layers to each other via openings in the braided layer.

The foregoing and other objects, features, and advantages of the invention will become more apparent from the following detailed description, which proceeds with reference to the accompanying figures.

### BRIEF DESCRIPTION OF THE DRAWINGS

FIG. 1 is a perspective view of a prosthetic heart valve, according to one embodiment.
FIG. 2A is a perspective view of a prosthetic heart valve, according to another embodiment.
FIG. 2B is a perspective view of the prosthetic valve of FIG. 2A with the components on the outside of the frame shown in transparent lines for purpose of illustration.
FIG. 3 is a perspective view of a delivery device for a prosthetic heart valve, according to an embodiment.
FIG. 4 is a cross-sectional view of an embodiment of a distal end portion of the delivery device of FIG. 3.
FIG. 5 is a side view of an assembly including a prosthetic valve crimped on a balloon mounted on a distal end portion of a balloon catheter, according to one embodiment.
FIG. 6 is a perspective view of a stepped pull ring, according to an embodiment.
FIG. 7A is a side view of the stepped pull ring of FIG. 6.
FIG. 7B is an end view of the stepped pull ring of FIG. 6.
FIG. 7C is a cross-sectional view of the stepped pull ring taken along line 7C-7C of FIG. 7B.
FIG. 8 is an enlarged perspective view of a cross-section of the distal end portion of an outer shaft of a delivery device showing a pull ring and an outer layer of the shaft removed for purposes of illustration, according to one embodiment.
FIG. 9 is another perspective view of the cross-section of the distal end portion of the outer shaft of FIG. 8, showing the outer layer of the shaft.
FIG. 10 is a cross-sectional view of the distal end portion of the outer shaft of FIG. 9.
FIG. 11 is a cross-sectional view of the distal end portion of the outer shaft of FIG. 10, taken along line 11-11 of FIG. 10.
FIG. 12 is a cross-sectional view of the distal end portion of the outer shaft of FIG. 10, taken along line 12-12 of FIG. 10.
FIG. 13A is a flowchart illustrating a method for making the shaft of FIGS. 8-12, according to one embodiment.
FIG. 13B is a flowchart illustrating a method for making the shaft of FIGS. 8-12, according to another embodiment.
FIG. 13C is a flowchart illustrating a method for making the shaft of FIGS. 8-12, according to another embodiment.
FIG. 14 is a cross-section of an extruded inner layer that can be used in forming the shaft of FIGS. 8-12.
FIG. 15 is an enlarged perspective view of a cross-section of the distal end portion of an outer shaft of a delivery device, according to another embodiment.
FIG. 16 is a cross-sectional view of the distal end portion of a delivery device incorporating the shaft of FIG. 15, according to one embodiment.
FIG. 17 is a side view of the distal end portion of the delivery device shown in FIG. 16.

### DETAILED DESCRIPTION

Described herein are examples of a steerable delivery apparatus (sometimes referred to as a steerable catheter) that can be used to navigate a subject's vasculature to deliver an implantable, expandable medical device (e.g., a prosthetic heart valve), tools, agents, or other therapy to a location within the body of a subject. Examples of procedures in which the steerable catheters are useful include neurological, urological, gynecological, fertility (e.g., in vitro fertilization, artificial insemination), laparoscopic, arthroscopic, transesophageal, transvaginal, transvesical, transrectal, and procedures including access in any body duct or cavity. Particular examples include placing implants, including stents, grafts, embolic coils, and the like; positioning imaging devices and/or components thereof, including ultrasound transducers; and positioning energy sources, for example, for performing lithotripsy, RF sources, ultrasound emitters, electromagnetic sources, laser sources, thermal sources, and the like.

The disclosed apparatus comprises a pull ring assembly with a stepped pull ring for fixing the distal end of a steering member, such as a pull wire or cable, to a distal end of a shaft of the delivery apparatus. In certain embodiments, the stepped pull ring has a proximal region with a larger diameter and a distal region with a smaller diameter and an intermediate transition region with an opening through which the distal end portion of the steering member can extend. In certain embodiments, the shaft can include a braided layer having a distal end portion extending into the larger proximal region of the pull ring.

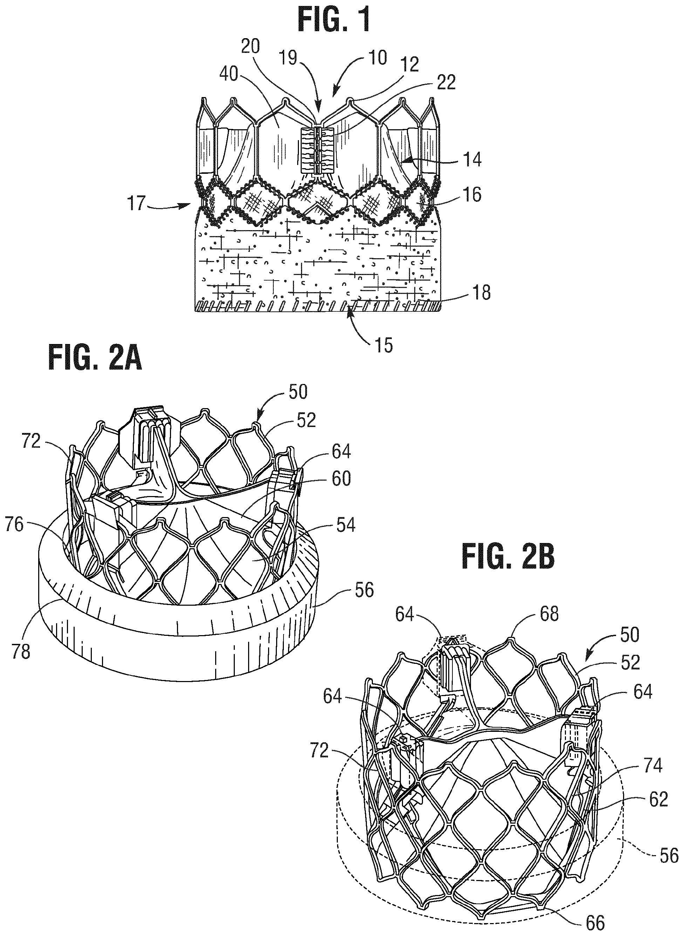

FIG. 1 shows a prosthetic heart valve 10, according to one embodiment. The illustrated prosthetic valve is adapted to be implanted in the native aortic annulus, although in other embodiments it can be adapted to be implanted in the other native annuluses of the heart (e.g., the pulmonary, mitral, and tricuspid valves). The prosthetic valve can also be adapted to be implanted in other tubular organs or passageways in the body. The prosthetic valve 10 can have four main components: a stent or frame 12, a valvular structure 14, an inner skirt 16, and a perivalvular outer sealing member or outer skirt 18. The prosthetic valve 10 can have an inflow end portion 15, an intermediate portion 17, and an outflow end portion 19.

The valvular structure 14 can comprise three leaflets 40, collectively forming a leaflet structure, which can be arranged to collapse in a tricuspid arrangement, although in other embodiments there can be greater or fewer number of leaflets (e.g., one or more leaflets 40). The leaflets 40 can be secured to one another at their adjacent sides to form commissures 22 of the leaflet structure 14. The lower edge of valvular structure 14 can have an undulating, curved scalloped shape and can be secured to the inner skirt 16 by sutures (not shown). In some embodiments, the leaflets 40 can be formed of pericardial tissue (e.g., bovine pericardial tissue), biocompatible synthetic materials, or various other suitable natural or synthetic materials as known in the art and described in U.S. Patent No. 6,730,118, which is incorporated by reference herein.

The frame 12 can be formed with a plurality of circumferentially spaced slots, or commissure windows 20 that are adapted to mount the commissures 22 of the valvular structure 14 to the frame. The frame 12 can be made of any of various suitable plastically-expandable materials (e.g., stainless steel, etc.) or self-expanding materials (e.g., nickel titanium alloy (NiTi), such as nitinol), as known in the art. When constructed of a plastically-expandable material, the frame 12 (and thus the prosthetic valve 10) can be crimped to a radially collapsed configuration on a delivery catheter and then expanded inside a patient by an inflatable balloon or equivalent expansion mechanism. When constructed of a self-expandable material, the frame 12 (and thus the prosthetic valve 10) can be crimped to a radially collapsed configuration and restrained in the collapsed configuration by insertion into a sheath or equivalent mechanism of a delivery catheter. Once inside the body, the prosthetic valve can be advanced from the delivery sheath, which allows the prosthetic valve to expand to its functional size.

Suitable plastically-expandable materials that can be used to form the frame 12 include, without limitation, stainless steel, a biocompatible, high-strength alloys (e.g., a cobalt-chromium or a nickel-cobalt-chromium alloys), polymers, or combinations thereof. In particular embodiments, frame 12 is made of a nickel-cobalt-chromium-molybdenum alloy, such as MP35N° alloy (SPS Technologies, Jenkintown, Pennsylvania), which is equivalent to UNS R30035 alloy (covered by ASTM F562-02). MP35N^{®} alloy/UNS R30035 alloy comprises 35% nickel, 35% cobalt, 20% chromium, and 10% molybdenum, by weight. Additional details regarding the prosthetic valve 10 and its various components are described in WIPO Patent Application Publication No. WO 2018/222799, which is incorporated herein by reference.

FIG. 2A is a perspective view of a prosthetic heart valve 50, according to another embodiment. The valve 50 can have three main components: a stent or frame, 52, a valvular structure 54, and a sealing member 56. FIG. 2B is a perspective view of the prosthetic valve 50 with the components on the outside of the frame 52 (including the sealing member 56) shown in transparent lines for purposes of illustration.

Like the valvular structure 14 of FIG. 1, the valvular structure 54 can comprise three leaflets 60, collectively forming a leaflet structure, which can be arranged to collapse in a tricuspid arrangement. Each leaflet 60 can be coupled to the frame 52 along its inflow edge 62 (the lower edge in the figures; also referred to as "cusp edges") and at commissures 64 of the valvular structure 54 where adjacent portions of two leaflets are connected to each other. A reinforcing element (not shown), such as a fabric strip, can be connected directly to the cusp edges of the leaflets and to the struts of the frame to couple the cusp edges of the leaflets to the frame.

Similar to the frame 12 of FIG. 1, the frame 52 can be made of any of various suitable plastically-expandable materials or self-expanding materials, as known in the art and described above. The frame 52 in the illustrated embodiment comprises a plurality of circumferentially extending rows of angled struts 72 defining rows of cells, or openings, 74 of the frame. The frame 52 can have a cylindrical or substantially cylindrical shape having a constant diameter from an inflow end 66 to an outflow end 68 of the frame as shown, or the frame can vary in diameter along the height of the frame, as disclosed in U.S. Patent 9,155,619, which is incorporated herein by reference.

The sealing member 56 in the illustrated embodiment is mounted on the outside of the frame 52 and functions to create a seal against the surrounding tissue (e.g., the native leaflets and/or native annulus) to prevent or at least minimize paravalvular leakage. The sealing member 56 can comprise an inner layer 76 (which can be in contact with the outer surface of the frame 52) and an outer layer 78. The sealing member 56 can be connected to the frame 52 using suitable techniques or mechanisms. For example, the sealing member 56 can be sutured to the frame 52 via sutures that can extend around the struts 72 and through the inner layer 76. In alternative embodiments, the inner layer 76 can be mounted on the inner surface of the frame 52, while the outer layer 78 is on the outside of the frame 52.

The outer layer 78 can be configured or shaped to extend radially outward from the inner layer 76 and the frame 52 when the prosthetic valve 50 is deployed. When the prosthetic valve is fully expanded outside of a patient's body, the outer layer 78 can expand away from the inner layer 76 to create a space between the two layers. Thus, when implanted inside the body, this allows the outer layer 78 to expand into contact with the surrounding tissue.

Additional details regarding the prosthetic valve 50 and its various components are described in U.S. Patent Publication No. 2018/0028310, which is incorporated herein by reference.

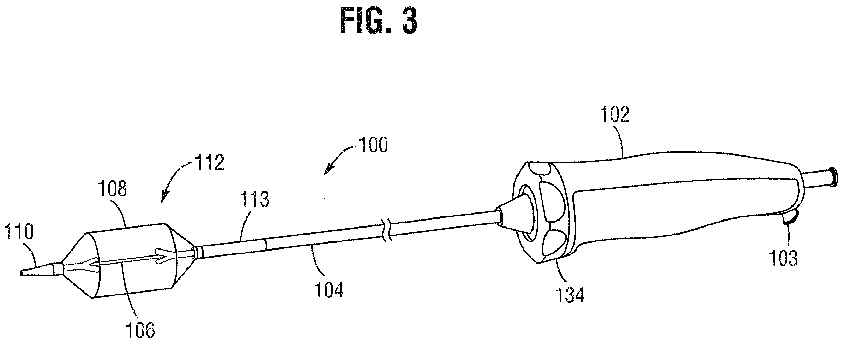

FIGS. 3-5 show various embodiments and components of a delivery system, such as a transcatheter delivery system, including a delivery apparatus (which, in one embodiment, may be a balloon catheter). FIG. 3 shows a delivery device (e.g., apparatus) 100, according to an embodiment, that can be used to implant an expandable prosthetic heart valve (e.g., heart valve 10 or 50), or another type of expandable prosthetic medical device (such as a stent). In some embodiments, the delivery device 100 is specifically adapted for use in introducing a prosthetic valve into a heart.

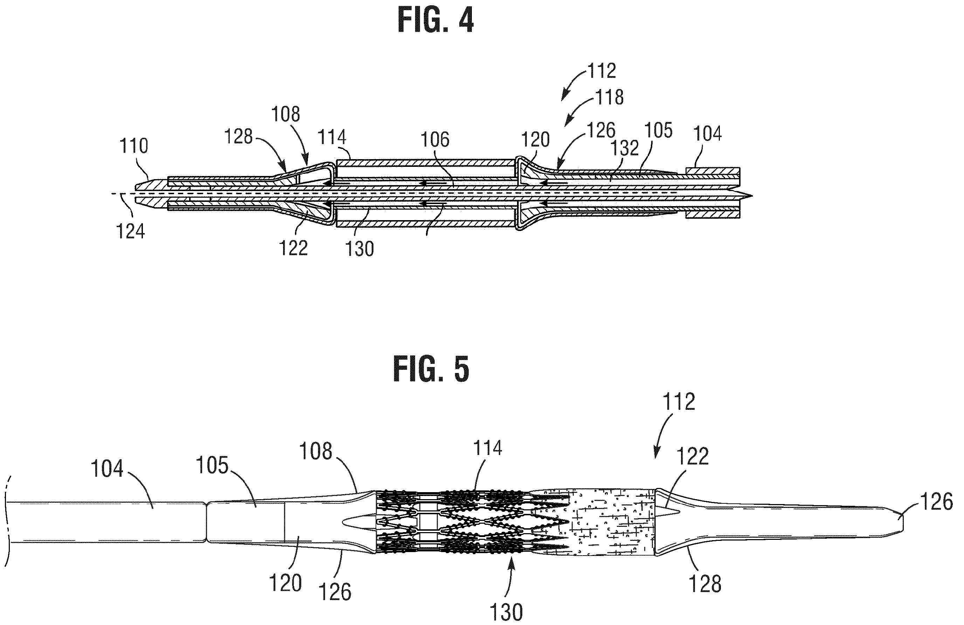

Referring to FIG. 3, the delivery device 100 in the illustrated embodiment is a balloon catheter comprising a handle 102, a steerable, outer shaft 104 extending from the handle 102, an intermediate shaft 105 (see FIG. 4) extending from the handle 102 coaxially through the steerable outer shaft 104, and an inner shaft 106 (FIG. 4) extending from the handle 102 coaxially through the intermediate shaft 105 and the outer shaft 104. The delivery device 100 further comprises an inflatable balloon 108 extending from a distal end of the intermediate shaft 105, and a nosecone 110 arranged at a distal end of the delivery device 100. A distal end portion 112 of the delivery device 100 includes the balloon 108, the nosecone 110, and a balloon shoulder assembly. A prosthetic medical device, such as a prosthetic heart valve may be mounted on a valve retaining portion of the balloon 108, as described further below with reference to FIG. 4. The balloon shoulder assembly maintains the prosthetic heart valve or other medical device at a fixed position on the balloon 108 during delivery through the patient's vasculature.

The handle 102 can include a steering mechanism configured to adjust the curvature of the steerable distal end portion 113 of the outer shaft 104 (herein also referred to as the steerable section 113 of outer shaft 104). In one example embodiment, the steerable section of the outer shaft comprises about one-quarter of the overall length of the outer shaft. A steering member, such as a pull wire or a braided cable, is connected to an adjustment member provided in the handle to adjust the curvature of the steerable section upon rotation of the adjustment member. In the illustrated embodiment, the handle 102 includes an adjustment member provided in the form of a rotatable knob 134. The rotatable knob 134 is operatively coupled to a proximal end portion the steering member inside the body of handle 102.

Rotating the knob 134 of the handle 102 is effective to increase or decrease the tension in the pull cable 202, thereby adjusting the curvature of the steerable section 113 of the outer shaft. By adjusting the curvature of the steerable section, an operator can guide the delivery device through the patient's vasculature to a selected site of valve deployment. In one specific example, the curvature of the steerable section is adjusted when navigating the delivery device through the aortic arch to deliver a prosthetic heart valve.

Shafts 102, 104, 105 (FIG. 3) can be formed from any of various suitable materials, such as nylon, braided stainless steel wires, or a polyether block amide. The shafts 102, 104, 105 can have also longitudinal sections formed from different materials in order to vary the flexibility of the shafts along their lengths.

In particular embodiments, the steerable section of the outer shaft is substantially straight in its non-deflected state. In other embodiments, the steerable section is slightly curved in its non-deflected state. In further embodiments, when fully deflected, the steerable section conforms to the shape of the aortic arch.

FIG. 4 shows an embodiment of the distal end portion 112 of the delivery device 100. As shown in FIG. 4, the delivery device 100 is configured to mount a prosthetic valve (e.g., prosthetic heart valve) 114 in a crimped state over the balloon 108 for insertion of the delivery device 100 and prosthetic valve 114 into a patient's vasculature.

As shown in FIG. 4, at a proximal end of the distal end portion 112, the inner shaft 106 extends distally beyond the steerable shaft 104 and the intermediate shaft 105 and through the balloon 108. The balloon 108 can be supported on a balloon shoulder assembly 118. The balloon shoulder assembly 118 includes a proximal shoulder 120 connected to a distal end of the intermediate shaft 105 and a distal shoulder 122 mounted on the inner shaft 106. The balloon 108 includes a proximal end portion 126 surrounding and/or folded over the proximal shoulder 120 and a distal end portion 128 surrounding and/or folded over the distal shoulder 122. In some embodiments, the proximal end portion 126 of the balloon 108 may be secured to the outer surface of the intermediate shaft 105. In some embodiments, the distal end portion 128 of the balloon 108 may be secured to the outer surface of the nosecone 110, which can be mounted on or coupled to the inner shaft 106.

In the illustrated embodiment, the nosecone 110 and the distal shoulder 122 can be a one-piece or unitary component, that is, the nosecone 110 is a distal portion of the unitary component and the distal shoulder 122 is a proximal portion of the unitary component. In other embodiments, the nosecone 110 and the distal shoulder 122 can be separate components, and each can be mounted on the inner shaft 106 next to each other or at axially spaced locations.

The proximal shoulder 120 and the distal shoulder 122 are spaced apart from one another, in an axial direction relative to a central longitudinal axis 124 of the delivery device 100. As a result, the balloon 108 defines a valve-retaining portion 130 in the space that separates the proximal shoulder 120 and the distal shoulder 122 (e.g., between flared ends of the proximal shoulder 120 and the distal shoulder 122). As shown in FIG. 4, the prosthetic valve 114 can be crimped onto the valve retaining portion 130 of the balloon 108, between the proximal shoulder 120 and the distal shoulder 122, thereby preventing or reducing axial movement of the prosthetic valve 114 relative to the balloon 108 during insertion of the delivery device 100 into the patient and delivery of the prosthetic valve 114 to the target implantation site.

The outer diameter of the inner shaft 106 can be sized such that an annular space 132 is defined between the inner shaft 106 and the intermediate shaft 105 along the entire length of the intermediate shaft 105. The annular space 132 may be fluidly coupled to one or more fluid passageways of the delivery device 100 which can be fluidly connectable to a fluid source (e.g., a syringe) that can inject an inflation fluid (e.g., saline) into the delivery device. In this way, fluid from the fluid source can flow through the one or more fluid passageways, through the annular space 132, and into the balloon 108 to inflate the balloon 108 and expand and deploy the prosthetic valve 114. For example, the handle 102 can have a fluid port 103 (see FIG. 3) configured to be coupled to the fluid source. In use, inflation fluid from the fluid source can be injected into the fluid port 103, through one or more fluid passageways in the handle 102, through the annular space 132, and into the balloon 108.

FIG. 4 illustrates the flow of fluid (indicated by arrows 109) through the annular space 132 and through passages in the proximal shoulder 120 and distal shoulder 122. The fluid can then flow into the proximal and distal end portions 126, 128 of the balloon 108 to expand the valve 114. Further details of the balloon shoulder assembly, the steering mechanism, and other components of the delivery device are disclosed in U.S. Patent Nos. 7,780,723; 9,061,119; 9,339,384; and U.S. Publication No. 2017/0065415, which are incorporated herein by reference.

FIG. 5 shows a side view of an exterior of the distal end portion 112 of the delivery device 100, including the prosthetic valve 114 crimped on the balloon 108 mounted on the distal end portion 112 of the balloon catheter. The balloon shoulder assembly (described above at FIG. 4) including the proximal shoulder 120 and distal shoulder 122 supports the balloon 108 thereon. As shown in FIG. 5, the balloon 108 includes the proximal end portion 126 surrounding and/or folded over the proximal shoulder 120, the distal end portion 128 surrounding and/or folded over the distal shoulder 122, and the valve retaining portion 130 located between the proximal end portion 126 and the distal end portion 128. The prosthetic valve 114 is crimped to the balloon catheter, on and around the valve retaining portion 130.

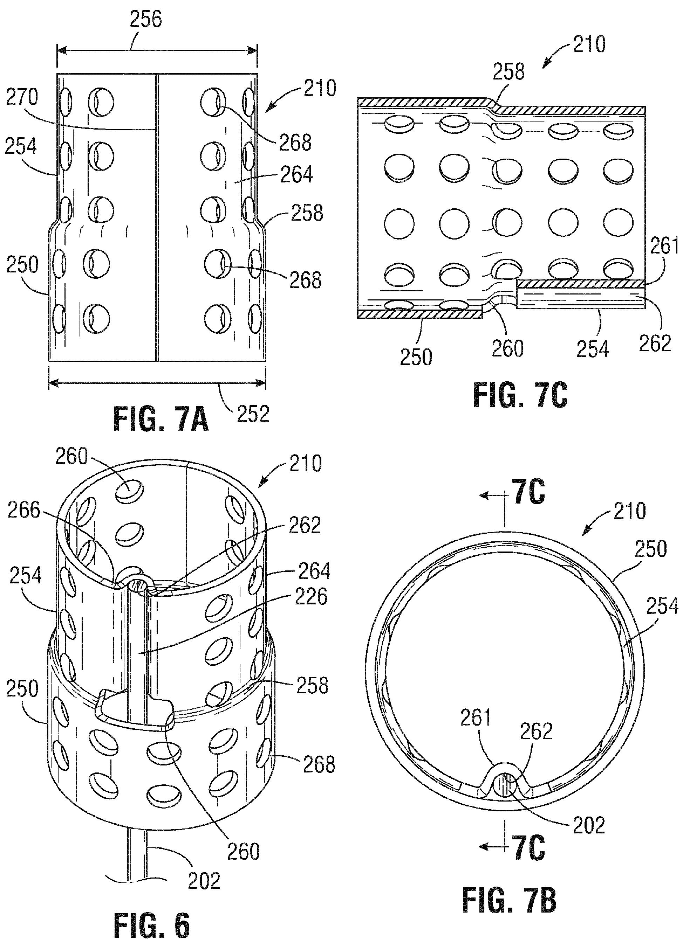

FIGS. 6-12 show the construction of the distal end portion of steerable outer shaft 200 that includes a steering member in the form of a pull cable 202 affixed to a pull ring 210. The pull cable 202 can comprise a plurality of braided wires or filaments, which can be made of any of various materials, including any of various metals or metal alloys (e.g., stainless steel), carbon fibers and/or polymer fibers. Alternatively, the steering member can comprise a single wire, such as a single metal wire. The outer shaft 200 can be implemented in the delivery apparatus 100 in place of the outer shaft 104. It should be understood that the shaft 200 need not be the outermost shaft of a delivery apparatus and instead can be an inner shaft or an intermediate shaft of a delivery apparatus, such as delivery apparatus 100.

Moreover, the shaft 200 can be implemented in other types of delivery apparatuses or other catheter devices that have steering mechanisms. For example, in some embodiments, the shaft 200 can be implemented in the delivery apparatus disclosed U.S. Provisional Application No. 63/138,890, filed January 19, 2021, which is incorporated herein by reference and which discloses another example of a delivery apparatus having a balloon for deploying a prosthetic valve. In other embodiments, the shaft 200 can be the steerable shaft of a delivery apparatus for a self-expandable prosthetic heart valve, such as disclosed in U.S. Publication No. 2019/0008640, U.S. Publication No. 2014/0343670, U.S. Publication No. 2012/0239142, or U.S. Publication No. 2010/0049313, which are incorporated herein by reference. In other embodiments, the shaft 200 can be the steerable shaft of a delivery apparatus for a mechanically-expandable prosthetic heart valve, such as disclosed in U.S. Application No. 62/945,039, which is incorporated herein by reference.

The steerable shaft 200 also can be implemented in delivery apparatuses that are used to deliver and implant medical devices other than prosthetic heart valves, such as leaflet repair devices that are implanted on one or more native heart valve leaflets, or implantable devices that are implanted within other organs of the body as previously described. Further, in other embodiments, the shaft 200 can be implemented in a steerable catheter, which can be configured for performing any various medical procedures as previously described, including, but not limited to any of various neurological, urological, gynecological, fertility (e.g., in vitro fertilization, artificial insemination), laparoscopic, arthroscopic, transesophageal, transvaginal, transvesical, or transrectal procedures.

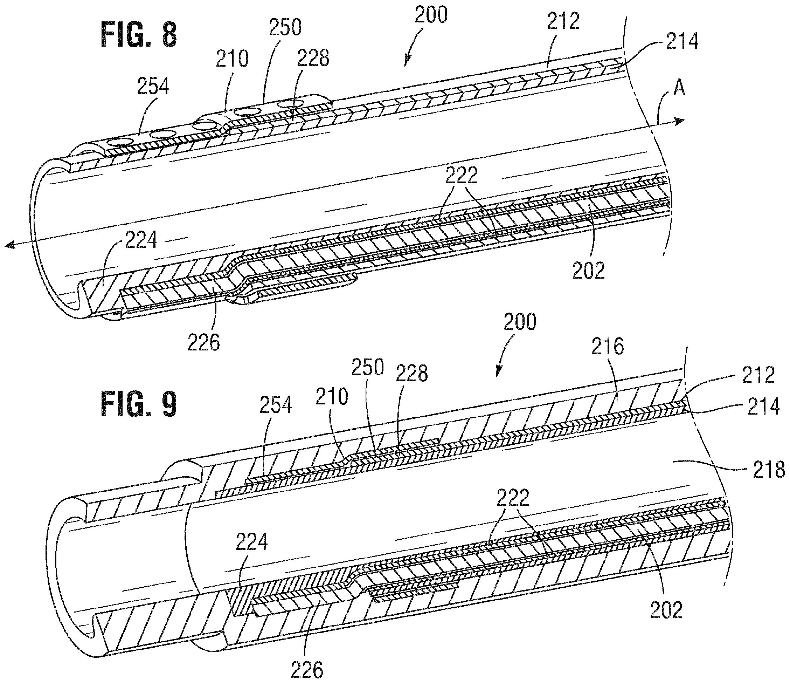

Referring to FIGS. 8 and 9, the shaft 200 in the illustrated embodiment includes a braided layer 212 disposed between an inner polymeric later 214 and an outer polymer layer 216. The braided layer 212 can impart flexibility, tensile strength, torque transmission, and stiffness to the outer shaft, while preventing or minimizing kinking. In certain embodiments, the braided layer 212 is formed from metal or polymeric filaments (e.g., stainless steel filaments) braided together. The braided layer 212 can extend the length of the outer shaft 200 from its proximal end at the handle 102 to the pull ring 210. Further details regarding the construction of the braided layer are disclosed in U.S. Publication No. 2017/0273787, which is incorporated herein by reference. In alternative embodiments, the braided layer 212 can be replaced with a polymeric or metal coil (e.g., a stainless steel coil).

The inner and outer layers 214, 216 can be formed from any of various suitable polymers, such as nylon, a polyether block amide (e.g., PEBAX^{®}, PEBA, VESTAMID^{®}), polyethylene or polytetrafluoroethylene (e.g., Teflon^{®}) or various other thermoplastics. The outer layer 216 may be made of the same polymeric material as the inner polymeric layer, or an alternate material (e.g., a material that is more flexible or more stiff than the material of the inner polymeric layer).

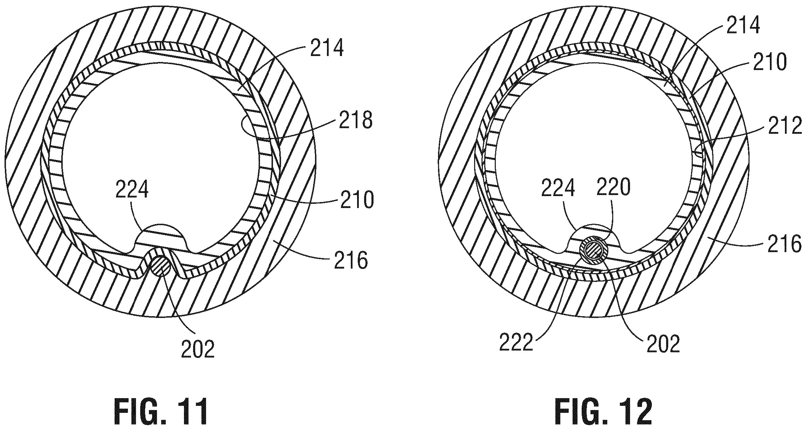

The inner layer 214 defines a main lumen 218 sized to receive one or more additional shafts of the delivery apparatus (e.g., the intermediate shaft 105 and the inner shaft 106 of the delivery apparatus 100). The inner layer 214 in the illustrated embodiment also defines a steering-member lumen 220 that is radially offset from a central longitudinal axis A of the shaft 200. The steering-member lumen 220 (as best shown in FIG. 12) is sized to receive the pull cable 202, which extends through the entire length of the lumen 220. The steering-member lumen 220 can be lined with a tubular liner 222, which can extend the entire length of the lumen 220. The liner 222 can be made of a relatively low friction polymeric material, such as polytetrafluoroethylene or a similar material, to minimize sliding friction between the pull cable 202 and the surrounding inner surface of the lumen. In some embodiments, the outer surface of the liner is etched to improve the thermal bond adhesion of the liner 222 to the inner layer 214. This thermal bond prevents or minimizes sliding of the liner 222 during pull cable actuation. In some embodiments, the inner surface of the main lumen 218 similarly can be covered with a relatively low friction polymeric material, such as polytetrafluoroethylene or a similar material, if another shaft (e.g., shaft 105) extends through the main lumen 218.

The pull cable 202 has a distal end portion 226 affixed to the pull ring 210, as further described below. The proximal end portion of the pull cable 202 is operatively connected to an adjustment mechanism on the handle of the delivery apparatus (e.g., the knob 134 of the delivery apparatus 100), which adjustment mechanism is configured to adjust the tension of the pull cable 202, thereby adjusting the curvature of the shaft 200. Various mechanisms may be used to couple the proximal end portion of the pull cable 202 the knob 134 of the delivery apparatus 100. In one specific embodiment, the proximal end portion of the pull cable 202 is secured to a slidable nut (not shown) inside the handle 102. The nut can have external threads that mate with internal threads of the knob 134. The nut is restricted from rotating with the knob 134 such that rotation of the knob (clockwise and counterclockwise) produces axial movement of the nut (in the proximal and distal directions) within the handle.

Since the distal end portion 226 of the pull cable 202 is fixed to the pull ring 210, the longitudinal motion of the slide nut via rotation of the knob changes the tension in the pull cable. The adjustment knob 134 can be turned in one direction to apply tension to the pull cable 202, and can be turned in the opposite direction to release tension thereupon. In some embodiments, the knob 134 is turned clockwise to apply tension, while in other embodiments, counter-clockwise rotation applies the tension. In any case, when tension is increased, the pull cable increases the curvature of the distal end portion of the shaft 200 (and therefore the distal end portion of the delivery apparatus 100). Conversely, when tension in the pull cable is decreased or released, the resiliency of the distal portion of the shaft causes the distal portion to return to its non-flexed configuration. In its non-flexed configuration (in the absence of pull-cable forces), the distal portion can be substantially straight (as shown in FIG. 3) or can be curved.

FIGS. 6 and 7A-C show details of the pull ring 210. As shown, the pull ring 210 in the illustrated embodiment comprises a proximal segment 250 having a first diameter 252 and a distal segment 254 having a second diameter 256 smaller than the first diameter. In one representative embodiment, the first diameter 252 is about .140 inch and the second diameter 256 is about .129 inch. In the illustrated embodiment, a length of the proximal segment 250 is shorter than a length of the distal segment 254. In another embodiment, a length of the proximal segment is longer than a length of the distal segment. In still another embodiment, the length of the proximal and distal segments are equal or substantially equal. In one representative embodiment, the length of the proximal segment 250 is about .100 inch and the length of the distal segment is about .100 inch. When assembled in the shaft 200, a distal end portion 228 of the braided layer 212 extends into the proximal segment 250 (see FIGS. 8-10) and terminates short of the distal segment 254.

The pull ring 210 can further comprise a transition section 258 formed between the proximal segment 250 and the distal segment 254. The transition section 258 can be formed with an opening 260. The opening 260 can be provided on only one side of the transition section 258 as shown, such that the remainder of the transition section 258 forms a continuous connection between the proximal segment 250 and distal segment 254. In other words, the proximal segment and the distal segment are discontinuous from each other only at the location of the opening 260, as best shown in FIG. 6 and 7C. The opening 260 may have any suitable shape. In the illustrated embodiment, opening 260 is trapezoid in shape. In other embodiments, the opening 260 may be circular, oval, elliptical, rectangular, oblong, etc. The opening 260 is sized to be large enough to receive the pull cable 202 therethrough.

The opening 260 can be in communication with a groove 262 formed on an inward, V-shaped projection 261 of the distal segment 254 of the pull ring 210. The groove 262 can extend along an entire length of the distal segment 254 such that when the pull cable 202 is received therein, the distal end portion 226 of the pull cable 202 can extend to the distal-most end 266 of the pull ring 210. Thus, the distal end portion 226 of the pull cable 202 extends through the proximal segment 250 of the pull ring, then through the opening 260, and into the groove 262. Further, the distal end portion 226 can be affixed to the distal segment 254 of the pull ring, such as by welding, crimping, an adhesive and/or mechanical fasteners. As described with reference to FIGS. 8-9, the pull ring 210 can be embedded between the inner layer 214 and the outer layer 216 of the shaft 200, which fixes the position of the pull ring 210 at a fixed location along the distal portion of the shaft 200. As a result, the distal end portion 226 of the pull cable 202 is secured at fixed location relative to the distal end portion of the shaft 200.

In the illustrated embodiment, the surface of both the proximal segment 250 and the distal segment 254 is perforated with holes 268. The holes can be distributed evenly throughout the surface of both the proximal and distal segments. In other embodiments, the holes may be distributed unevenly, such as with the proximal segment being more perforated than the distal segment, or the distal segment being more perforated than the proximal segment. While the depicted embodiment shows the perforations as circular holes 268, in other embodiments, the perforations may be shaped and sized differently. During assembly, the holes 268 allow the material forming inner and outer layers 214, 216, respectively, to bond to each other through the holes 268, which assists in fixing the pull ring in place between the layers 214, 216.

In one example, the pull ring 210 is manufactured through a stamping process and then seam welded, generating a seam 270. For example, with reference to the perforated embodiment of FIGS. 6 and 7A-C, starting with a flat sheet of material (e.g., sheet metal), holes 268 and opening 260 are punched into the sheet, or cut with a laser. Then, the groove 262 and the cylindrical, stepped shape of the pull ring is created using stamping and forming processes, as is known in the art. The adjacent longitudinal edges of the fully formed pull ring can be welded together to form the seam 270. In alternative embodiments, the pull ring 210 can be made from other materials and/or via manufacturing techniques. For example, the pull ring 210 can be molded (e.g., injection molded) and made from a suitable polymer, such as PLEXAR^{®} resin, PEBAX^{®}, PTFE, FEP or other suitable materials.

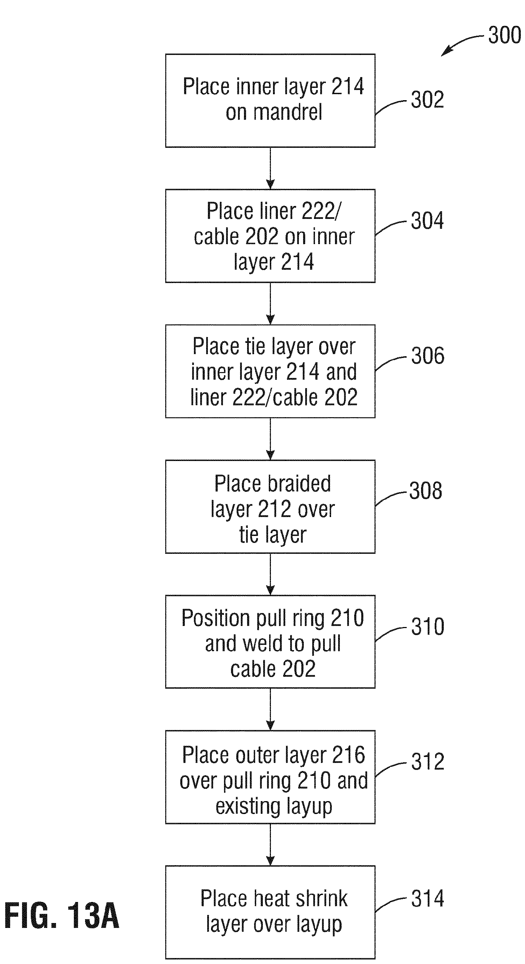

FIG. 13A shows a flow chart of a method 300 for manufacturing the shaft 200, according to one embodiment. As shown in FIG. 13A, at block 302, the inner polymeric layer 214 can be placed on a mandrel, which can have a longitudinal groove. The pull cable 202 can be placed along the outside of the inner layer 214 within the groove of the mandrel. When a liner 222 is used, an assembly comprising the pull cable 202 encased in the liner 222 can be placed along the outside of the inner layer 214 within the groove of the mandrel, as shown in block 304. The distal end portion 226 of the pull cable 202 can extend outwardly from the distal end of the liner 222, leaving the distal end portion 226 uncovered by the liner for later attachment to the pull ring 210.

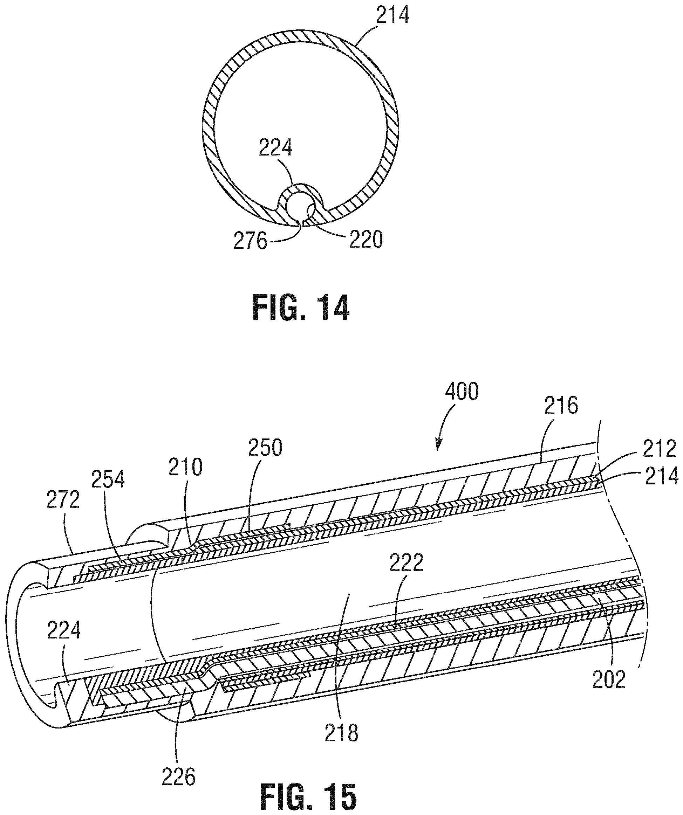

In some embodiments, the inner layer 214 can be extruded to its final or near final shape prior to placing the inner layer 214 on the mandrel. As shown in FIG. 14, the extruded inner layer 214 can be formed with the inward projection 224, the steering-member lumen 220, and slit or slot 276 in communication with the lumen 220 extending the length of the inner layer 214. The slit 276 allows the pull cable 202 and the liner 222 to be inserted into the lumen 220. In some embodiments, the extruded inner layer 214 can be extruded without the slit 276, in which case the slit 276 can be formed by cutting the inner layer lengthwise along the lumen 220. After forming the inner layer 214 and placing the pull cable 202 and the liner 222 in the lumen, the inner layer 214 can be positioned on the mandrel with the projection 224 aligned within the longitudinal groove of the mandrel.

In some embodiments, an optional polymeric tie layer can be placed over the liner 222/pull cable 202 and the inner layer 214, as shown at block 306. The tie layer can be made of a PLEXAR^{®} resin, PEBAX^{®}, PTFE, FEP or other suitable materials.

As shown at block 308, the braided layer 212 is then placed over the tie layer, leaving a distal section of the inner layer, the tie layer and the distal end portion 226 of the pull cable uncovered by the braided layer 212 for later placement of the pull ring 210. If a tie layer is not used, then the braided layer can be placed directly over the inner layer 214 and the liner 222/pull cable 202. In some embodiments, the braided layer 212 can be pre-formed (e.g., braided or woven into a tubular structure) and placed (e.g., pulled) over the underlying layers. In alternative embodiments, the braided layer 212 can be braided or woven around the underlying layers such that the braided layer is formed as it is placed over the underlying layers.

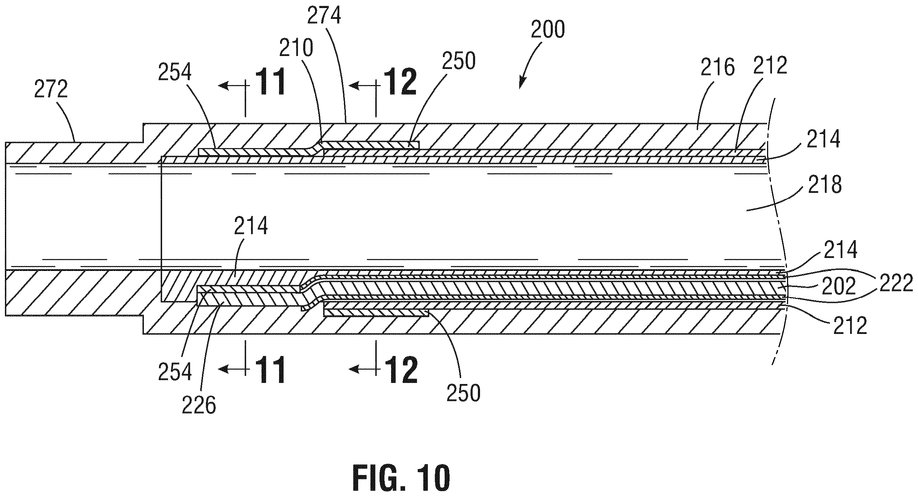

As shown at block 310, the pull ring 210 is then slid over the distal end portion of the layup comprising the inner layer 214, the liner 222/pull cable 202, the tie layer, and the braided layer 212 such that the larger proximal end portion 250 of the pull ring 210 extends over the braided layer 212 and the distal end portion 254 of the pull ring 210 extends over the tie layer with the distal end portion 226 of the pull cable extending through the opening 260 and into the groove 262 of the pull ring 210 (as shown in FIG. 10). The inward projection 261 of the pull ring can be aligned with the groove of the mandrel. The distal end portion 226 of the pull cable 202 can be affixed to the pull ring, such as by welding the distal end portion 226 of the cable to the surface of the distal end portion 254 of the pull ring within the groove 262. Alternatively, the distal end portion of the pull cable 202 can be affixed to the pull ring by mechanical means, such as by fixing an enlarged end component to the distal end of the cable 202. The end component has a larger width than that of the opening 260 to prevent the distal end of the cable from being pulled proximally through the opening 260. The end component can be, for example, a ferrule, a ring, or a sphere that has a larger diameter than that of the cable and is sized such that it cannot be pulled through the opening 260 in the proximal direction. In this manner, the distal end portion 226 of the cable 202 need not be securely bonded or fixed to the pull ring and instead can "float" relative to the pull ring as tension in the cable is adjusted but does not separate from the pull ring in the proximal direction. In alternative embodiments, although less desirable, a conventional pull wire can be used in placed of the pull cable.

As shown at block 312, the outer layer 216 can then be placed over the braided layer 212, the pull ring 210 and any remaining uncovered section along the distal end portion of the inner layer 214 and tie layer. As shown at block 314, the outer layer 216 can then be encased in a heat shrink material and heated. The heat causes the outer layer to flow through the openings 268 in the pull ring 210 and into and through the braided layer and into contact with the tie layer or the inner layer 214, forming a shaft having a laminate construction. The applied heat can also cause the inner layer 214 to flow and seal the slit 276 around the pull cable 202. If the inner layer 214 is initially a cylindrical layer, the inner layer can flow around the liner 222, thereby forming a steering member lumen 220 entirely within or substantially within the wall thickness of the inner layer 214.

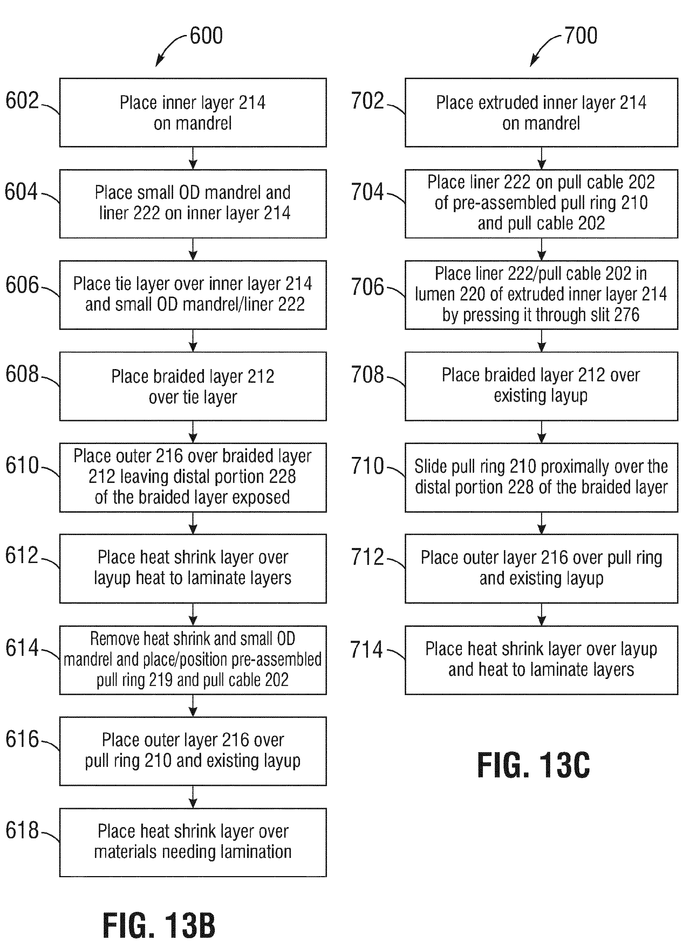

FIG. 13B shows a flow chart of a method 600 for manufacturing the shaft 200, according to another embodiment. As shown in FIG. 13B, at block 602, the inner polymeric layer 214 can be placed on a main mandrel, which can have a longitudinal groove. At block 604, an assembly comprising a liner 222 and a second, small diameter mandrel (instead of the pull cable) inside of the liner is placed along the inner layer 214, such as aligned with the longitudinal groove of the main mandrel. At block 606, a tie layer can be placed over the inner layer 214 and the liner 222/small diameter mandrel. At block 608, the braided layer 212 can be placed over the tie layer. At block 610, the outer layer 216 can be placed over the braided layer 212, leaving the distal end portion 228 of the braided layer exposed. At block 612, a heat shrink layer is placed over the previously formed layup, which is heated to laminate the layers. At block 614, the heat shrink layer is removed from the layup and the small diameter mandrel is removed from the liner 222, and an assembly comprising the pull ring 210 and the pull cable 202 (which can be preassembled or connected to each other, such as by welding or other techniques described above) is positioned on the layup. For example, the pull cable 202 can be inserted through the liner 222 and the pull ring 210 can be placed over the distal end portion 228 of the braided layer, such as shown in FIG. 8. At block 616, the distal end portion of the outer layer 216 or an additional piece of polymer for forming the distal end portion of the outer layer is placed over the pull ring 210. At block 618, a heat shrink layer is placed over the distal end portion of the outer layer and heated to laminate the layup along the distal end portion of the outer layer.

FIG. 13C shows a flow chart of a method 700 for manufacturing the shaft 200, according to another embodiment. As shown in FIG. 13C, at block 702, an extruded inner layer 214 (such as shown in FIG. 14), is placed on a mandrel. At block 704, a liner 222 is placed on a pull cable 202, which can have a pre-attached or preconnected pull ring 210. At block 706, the liner 222 and pull cable 202 can be placed within the lumen 220, such as by pressing these components through the slit 276. At block 708, the braided layer 212 can be placed over the inner layer 214. At block 710, the pull ring 210 can be slid proximally over the distal end portion 228 of the braided layer 212. At block 712, an outer layer 216 can be placed over the pull ring 210 and the previously formed layup. At block 714, a heat shrink layer can be placed over the outer layer and then heated to laminate the layup.

The methods and arrangements of the present disclosure provide several advantages over the prior art method described above. For example, the distal end portion 254 of the pull ring 210 need not extend over the braided layer 212, eliminating the need to cut an opening in the braided layer for the pull cable 202. Instead, as best shown in FIG. 10, the pull cable 202 extends distally beyond the distal end of the braided layer 212 into the groove 262 of the distal end portion 254 of the pull ring.

Moreover, the smaller diameter distal end portion 254 of the pull ring and its inward projection 261 brings the distal end portion 226 of the pull cable into close proximity to the groove of the mandrel, thereby reducing the distance the pull cable extends radially inwardly from the pull ring into the braided layer. This avoids canting of the pull ring when tension is applied to the pull cable 202 or during the heating process (when the shaft 200 is formed), which can cause the pull ring to perforate the main lumen 218. The length of the proximal end portion 250 of the pull ring can be sufficiently long to further resist any residual canting of the pull ring during the heating process or under a relatively high tensile force applied to the pull cable.

Further, although a conventional pull wire can be used, a pull cable 202 is advantageous because a cable is more flexible than a wire of a similar size and can easily form a curved transition section extending from the pull ring 210 into the lumen 220. The more flexible cable can better retain its curved shape along the transition section during the heating process or when tension is applied to the cable to avoid canting of the pull ring, whereas a stiffer wire tends to straighten along the transition section, which can cause canting of the pull ring.

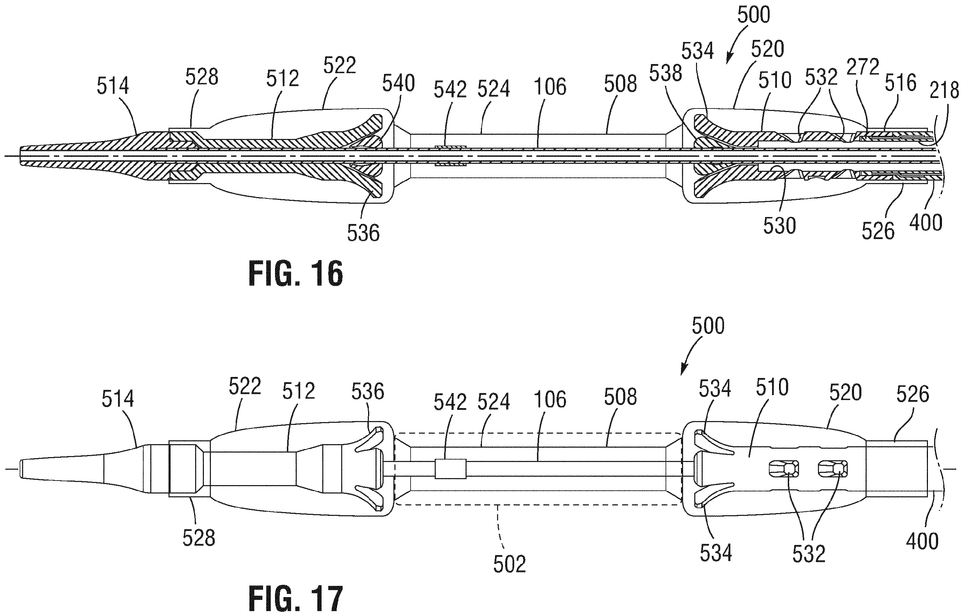

In some embodiments of the delivery device, as best shown in FIGS. 9 and 10, the shaft 200 can have a stepped configuration along a distal end portion thereof. In the depicted embodiment, the outer layer 216 has a distal section 272 of a smaller diameter than a section 274 extending proximally from the distal section 272. The presence of a stepped region having a smaller diameter can facilitate coupling of a delivery apparatus component to the distal end of the shaft 200. In addition, the stepped region can be sized such that, after coupling, the outer surface of the coupled component can be flush with the outer surface of the shaft 200. In one example, a proximal shoulder (e.g., a proximal shoulder 510; see FIG. 16) can be mounted on the distal section 274, as further described below.

FIG. 15 shows the distal end portion of a shaft 400, according to another embodiment, which can be incorporated in a delivery device, such as delivery device 100 or delivery device 500 (discussed below). The shaft 400 can be identical to the shaft 200 described above and can include a pull ring 210, a braided layer 212, an inner polymeric layer 214, an outer polymeric layer 216, a pull cable 202 (or a pull wire or another steering member) and a liner 222. The shaft 400 can be formed in the same manner as described above in connection with shaft 200, except that the pull ring 210 extends at least partially into the reduced diameter distal section 272 of the shaft 400.

As shown in FIG. 15, the majority of the length of the distal segment 254 of the pull ring 210 extends into the distal section 272 of the shaft 400. In some embodiments, the entirety of the distal segment 254 is positioned within the distal section 272 of the shaft 400. Positioning the pull ring 210 at least partially within the distal section 272 of the shaft 400 brings the pull ring 210 and the pull cable 202 into close proximity to the prosthetic valve (which is mounted distal to the distal section 272) to enhance the steerability of the distal end portion of the delivery device and control over the positioning of the prosthetic valve within the patient's vasculature.

FIGS. 16 and 17 show the distal end portion of a delivery device 500, according to one embodiment, that incorporates the shaft 400. The delivery device 500 also includes an inner shaft 106, but the intermediate shaft 105 of the delivery device 100 is omitted. Thus, in the illustrated embodiment, the delivery device 500 includes only two shafts. However, it should be understood that in alternative embodiments, additional shafts can be incorporated into the delivery device, such as an intermediate shaft 105 extending between the shafts 106, 400, or an additional shaft extending over the shaft 400 (in which case shaft 400 is not an outermost shaft of the delivery device).

The proximal end portions of the shaft 400 and the inner shaft 106 can be connected to a handle 102 (as shown in FIG. 3), which can have an adjustment knob 134 or similar adjustment mechanism for controlling the tension in the pull cable 202 and a fluid port 103. Since the intermediate shaft 105 is omitted, the main lumen 218 of the shaft 400 serves as an inflation lumen for conducting an inflation fluid from the fluid port 103 of the handle 102 into a balloon 508.

The delivery device 500 can include a proximal shoulder 510 mounted to the shaft 400, a distal shoulder 512 mounted to the inner shaft 106, and a nose cone 514 connected to and extending distally from the distal shoulder 512. As shown in FIG. 16, a proximal section 516 of the proximal shoulder 510 can be mounted on the distal section 272 of the shaft 400, such as by welding and/or an adhesive. The proximal section 516 can have an outer diameter that is equal to the outer diameter of the shaft 400 just proximal to the distal section 272 to provide a smooth transition between the shaft and the proximal shoulder.

The balloon 508 includes a proximal end portion 520 surrounding and/or folded over the proximal shoulder 510, a distal end portion 522 surrounding and/or folded over the distal shoulder 512, and an intermediate portion 524 for receiving a prosthetic valve 502 (which can be, e.g., valve 10 or 50). The proximal end portion 520 can include a leg portion 526 that is sealed against the proximal shoulder 510 and/or the shaft 400, such as with a suitable adhesive and/or welding. Similarly, the distal end portion 522 can include a leg portion 528 that is sealed against the distal shoulder 512 and/or the nose cone 514, such as with a suitable adhesive and/or welding.

The proximal shoulder 510 can be formed with an inner bore or lumen 530 in communication with the lumen 218 of the shaft 400 and one or more inflation ports or openings 532. In this manner, pressurized inflation fluid within lumen 218 can flow distally into the proximal shoulder 510 and then radially outwardly through the ports 532 into the proximal portion 520 of the balloon 508. Inflation fluid within the proximal portion 520 can then flow through the intermediate portion 524 and into the distal portion 522 of the balloon, thereby inflating the balloon 508 and expanding the prosthetic valve 502 mounted on the balloon.

The proximal shoulder 510 can include a flared distal section, which can comprise a plurality of wings or flared legs 534 defining gaps therebetween. Similarly, the distal shoulder 512 can include a flared proximal section, which can comprise a plurality of wings or flared legs 536 defining gaps therebetween. The flared sections help retain the prosthetic valve 502 on the intermediate section 524 of the balloon 508. The gaps between the legs 534, 536 allow the legs to resiliently collapse radially so that the shoulders 510, 512 can be inserted into the smaller diameter leg portions 526, 528 of the balloon during assembly. Additionally, the gaps between the legs 534, 536 promote the flow of inflation fluid through the balloon from the proximal end to the distal end of the balloon.

As shown in FIG. 16, the delivery device 500 can include a proximal hub 538 mounted on the inner shaft 106 within the legs 534 of the proximal shoulder 510 and a distal hub 540 mounted on the inner shaft 106 within the legs 536 of the distal shoulder 512. The hubs 538, 540 can be affixed to the inner shaft using various techniques and mechanisms, including an adhesive, thermal bonding, or over molding the hubs to the shaft. In some embodiments, one or both of the hubs 538, 540 form a sliding fit with the inner shaft 106. For example, in certain embodiments, the proximal hub 538 forms a sliding fit with the inner shaft 106, while the distal hub 540 is rigidly affixed to the inner shaft 106, such as with an adhesive, thermal bonding or over molding. When the curvature of the outer shaft 400 is adjusted, the hub 538promotes the transfer of the flex motion of the outer shaft 400 to the prosthetic valve 502 by keeping the inner shaft 106 concentric with the proximal shoulder 510. The hubs also minimize bending between the shoulders 510, 512 and the adjacent ends of the prosthetic valve to facilitate crossing the native aortic annulus.

The delivery device 500 also can include one or more radiopaque markers 542 mounted on the shaft 106 in the area within the intermediate portion 524 of the balloon. The markers 542 can be used for positioning the prosthetic valve 502 at a native annulus under fluoroscopy.

### General Considerations

For purposes of this description, certain aspects, advantages, and novel features of the embodiments of this disclosure are described herein. The described methods, systems, and apparatus should not be construed as limiting in any way. Instead, the present disclosure is directed toward all novel and non-obvious features and aspects of the various disclosed embodiments, alone and in various combinations and sub-combinations with one another. The disclosed methods, systems, and apparatus are not limited to any specific aspect, feature, or combination thereof, nor do the disclosed methods, systems, and apparatus require that any one or more specific advantages be present, or problems be solved.

Features, integers, characteristics, compounds, chemical moieties, or groups described in conjunction with a particular aspect, embodiment or example of the disclosure are to be understood to be applicable to any other aspect, embodiment or example described herein unless incompatible therewith. All of the features disclosed in this specification (including any accompanying claims, abstract, and drawings), and/or all of the steps of any method or process so disclosed, may be combined in any combination, except combinations where at least some of such features and/or steps are mutually exclusive. The disclosure is not restricted to the details of any foregoing embodiments. The disclosure extends to any novel one, or any novel combination, of the features disclosed in this specification (including any accompanying claims, abstract, and drawings), or to any novel one, or any novel combination, of the steps of any method or process so disclosed.

Although the operations of some of the disclosed methods are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods, systems, and apparatus can be used in conjunction with other systems, methods, and apparatus.

As used herein, the terms "a," "an," and "at least one" encompass one or more of the specified element. That is, if two of a particular element are present, one of these elements is also present and thus "an" element is present. The terms "a plurality of' and "plural" mean two or more of the specified element.

As used herein, the term "and/or" used between the last two of a list of elements means any one or more of the listed elements. For example, the phrase "A, B, and/or C" means "A," "B," "C," "A and B," "A and C," "B and C," or "A, B, and C."

As used herein, the term "coupled" generally means physically coupled or linked and does not exclude the presence of intermediate elements between the coupled items absent specific contrary language.

In the context of the present application, the terms "lower" and "upper" are used interchangeably with the term's "inflow" and "outflow", respectively. Thus, for example, the lower end of the valve is its inflow end and the upper end of the valve is its outflow end.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

### Additional Examples of the Disclosed Technology

In view of the above described implementations of the disclosed subject matter, this application discloses the additional examples enumerated below. It should be noted that one feature of an example in isolation or more than one feature of the example taken in combination and, optionally, in combination with one or more features of one or more further examples are further examples also falling within the disclosure of this application.

Example 1. A delivery apparatus for an implantable medical device, comprising:
a handle;
a shaft extending distally from the handle, the shaft having a steering-member lumen extending a length of the shaft;
a steering member extending through the steering-member lumen, the steering member having a proximal end portion and a distal end portion; and
a pull ring disposed along a distal end portion of the shaft, the pull ring comprising a proximal segment having a first diameter and a distal segment having a second diameter smaller than the first diameter, wherein the distal end portion of the steering member is fixed to the distal segment of the pull ring;
wherein the handle comprises an adjustment mechanism operatively coupled to the proximal end portion of the steering member and configured to adjust tension in the steering member so as to adjust a curvature of the shaft.

Example 2. The delivery apparatus of any example herein, particularly Example 1, wherein the pull ring further comprises a transition section formed between the proximal segment and the distal segment with an opening which is in communication with a groove formed on an outer surface of the distal segment of the pull ring.

Example 3. The delivery apparatus of any example herein, particularly Example 2, wherein the distal end portion of the steering member extends through the proximal segment of the pull ring, the opening, and into the groove.

Example 4. The delivery apparatus of any example herein, particularly any one of Examples 1-3, wherein the shaft comprises a braided layer radially outward of the steering-member lumen, wherein the braided layer has a distal end portion that extends into the proximal segment of the pull ring.

Example 5. The delivery apparatus of any example herein, particularly Example 4, wherein the distal end portion of the steering member extends distally beyond a distal terminal end of the braided layer.

Example 6. The delivery apparatus of any example herein, particularly any one of Examples 4 or 5, wherein the shaft comprises an inner polymeric layer defining the steering-member lumen and an outer polymeric layer, wherein the inner polymeric layer is radially inward of the braided layer and the outer polymeric layer is radially outward of the braided layer.

Example 7. The delivery apparatus of any example herein, particularly Example 6, wherein the pull ring is embedded within the shaft between the inner and outer polymeric layers.

Example 8. The delivery apparatus of any example herein, particularly any one of Examples 1-7, wherein the steering member comprises a braided cable.

Example 9. The delivery apparatus of any example herein, particularly any one of Examples 1-7, wherein the steering member comprises a wire.

Example 10. The delivery apparatus of any example herein, particularly any one of Examples 1-9, wherein a length of the proximal segment is shorter than a length of the distal segment.

Example 11. The delivery apparatus of any example herein, particularly any one of Examples 1-10, wherein a surface of one or both of the proximal and distal segments of the pull ring is perforated.

Example 12. The delivery apparatus of any example herein, particularly any one of Examples 1-11, wherein the shaft is an outer shaft having a central lumen, the delivery apparatus further comprising an inner shaft extending coaxially through the central lumen of the outer shaft, and wherein the steering-member lumen is radially offset from a central axis of the central lumen.

Example 13. A steerable delivery apparatus for an implantable medical device, comprising:
a handle;
a shaft extending distally from the handle, the shaft having a steering-member lumen extending between a distal end portion and a proximal end portion of the shaft, and a braided layer radially outward of the steering-member lumen;
a steering member extending through the steering-member lumen, the steering member having a proximal end portion and a distal end portion; and
a pull ring coupled to the distal end portion of the shaft, the pull ring comprising a proximal segment having a first diameter and a distal segment having a second diameter smaller than the first diameter, wherein the distal end portion of the steering member is fixed to the distal segment of the pull ring, and wherein a distal end portion of the braided layer extends into the proximal segment of the pull ring and terminates at a location proximal to the distal segment of the pull ring;
wherein the handle comprises an adjustment mechanism operatively coupled to the proximal end portion of the steering member, wherein actuation of the adjustment mechanism varies tension in the steering member to vary a curvature of the shaft.

Example 14. The delivery apparatus of any example herein, particularly Example 13, wherein the pull ring further comprises a transition section formed between the proximal segment and the distal segment with an opening which is in communication with a groove formed on an outer surface of the distal segment of the pull ring, and wherein the distal end portion of the steering member extends through the proximal segment of the pull ring, the opening, and into the groove.

Example 15. The delivery apparatus of any example herein, particularly Example 14, wherein the transition section tapers from the proximal segment to the distal segment of the pull ring.

Example 16. The delivery apparatus of any example herein, particularly any one of Examples 13-15, wherein a distal terminal end of the steering member extends distally beyond a distal terminal end of the braided layer.

Example 17. The delivery apparatus of any example herein, particularly any one of Examples 13-16, wherein the shaft comprises:
an inner polymeric layer defining the steering-member lumen, the inner polymeric layer disposed radially inward of the braided layer; and
an outer polymeric layer disposed radially outward of the braided layer, and wherein the pull ring is embedded within the distal end portion of the shaft between the inner and outer polymeric layers.

Example 18. The delivery apparatus of any example herein, particularly any one of Examples 13-17, wherein a surface of one or both of the distal and proximal segments of the pull ring comprises perforations.

Example 19. The delivery apparatus of any example herein, particularly any one of Examples 13-18, wherein the steering member comprises a braided cable.

Example 20. The delivery apparatus of any example herein, particularly any one of Examples 13-18, wherein the steering member comprises a wire.

Example 21. The delivery apparatus of any example herein, particularly any one of Examples 13 or 16-20, wherein the distal end portion of the steering member extends through the proximal segment of the pull ring, radially outwardly through an opening in the pull ring, and alongside an outer surface of the distal segment of the pull ring.

Example 22. The delivery apparatus of any example herein, particularly Example 21, wherein the distal end portion of the steering member extends into a groove in the distal segment of the pull ring.

Example 23. The delivery apparatus of any example herein, particularly any one of Examples 13-22, wherein the shaft is first shaft having a central lumen, the delivery apparatus further comprising a second shaft extending coaxially through the central lumen of the first shaft.

Example 24. The delivery apparatus of any example herein, particularly Example 23, further comprising an inflatable balloon mounted on a distal end portion of the second shaft.

Example 25. A steerable delivery apparatus for an implantable medical device, comprising:
a handle;
a shaft extending distally from the handle, the shaft having a steering-member lumen extending between a distal end portion and a proximal end portion of the shaft, a first layer, a second layer disposed co-axially over the first layer, and a third layer disposed co-axially over the second layer;
a steering member extending through the steering-member lumen, the steering member having a proximal end portion and a distal end portion; and
a pull ring coupled to the distal end portion of the shaft, the pull ring comprising a proximal segment having a first diameter and a distal segment having a second diameter smaller than the first diameter, wherein the distal end portion of the steering member is fixed to the distal segment of the pull ring, and wherein a distal end portion of the second layer of the shaft extends into the proximal segment of the pull ring and terminates at a location proximal to the distal segment of the pull ring;
wherein the handle comprises an adjustment mechanism operatively coupled to the proximal end portion of the steering member, wherein actuation of the adjustment mechanism varies tension in the steering member to vary a curvature of the shaft.

Example 26. The delivery apparatus of any example herein, particularly Example 25, wherein the second layer of the shaft comprises a braided layer.

Example 27. The delivery apparatus of any example herein, particularly any one of Examples 25-26, wherein the first and third layers of the shaft are polymeric layers and the pull ring is embedded between the first and third layers.

Example 28. The delivery apparatus of any example herein, particularly any one of Examples 25-27, wherein the pull ring further comprises a transition section formed between the proximal segment and the distal segment with an opening which is in communication with a groove formed on an outer surface of the distal segment of the pull ring, and wherein the distal end portion of the steering member extends through the proximal segment of the pull ring, the opening, and into the groove.

Example 29. The delivery apparatus of any example herein, particularly Example 28, wherein the transition section tapers from the proximal segment to the distal segment of the pull ring.

Example 30. The delivery apparatus of any example herein, particularly any one of Examples 25-29, wherein a distal terminal end of the steering member extends distally beyond a distal terminal end of the second layer of the shaft.

Example 31. The delivery apparatus of any example herein, particularly any one of Examples 25-30, wherein a surface of one or both of the distal and proximal segments of the pull ring comprises perforations.

Example 32. The delivery apparatus of any example herein, particularly any one of Examples 25-31, wherein the steering member comprises a braided cable.

Example 33. The delivery apparatus of any example herein, particularly any one of Examples 25-31, wherein the steering member comprises a wire.

Example 34. A method of using a catheter device, the method comprising:
inserting a shaft of the catheter device into the body of a patient, the shaft comprising a steering-member lumen extending there-through, a steering member extending through the steering-member lumen, the steering member having a proximal end portion and a distal end portion, the distal end portion of the steering member fixed to a distal end portion of the shaft via a pull ring, the pull ring comprising a proximal segment having a first diameter and a distal segment having a second diameter, wherein the first diameter is larger than the second diameter; and
steering the shaft inside the body of the patient by actuating an adjustment mechanism on a handle of the device outside the body of the patient, wherein the adjustment mechanism is operatively coupled to the proximal end portion of the steering member and wherein the actuating includes adjusting tension in the steering member to adjust a curvature of the shaft.

Example 35. The method of any example herein, particularly Example 34, wherein the shaft comprises a braided layer radially outward of the steering-member lumen, , wherein a distal end portion of the braided layer extends into the proximal segment.

Example 36. The method of any example herein, particularly any one of Examples 34-35, wherein the distal end portion of the steering member extends through the proximal segment, an opening disposed between the proximal and distal segment, and into a groove formed on an outer surface of the distal segment.

Example 37. The method of any example herein, particularly any one of Examples 35-36, wherein the shaft comprises:
an inner polymeric layer defining the steering-member lumen, the inner polymeric layer disposed radially inward of the braided layer; and
an outer polymeric layer disposed radially outward of the braided layer, and wherein the pull ring is embedded within the distal end portion of the shaft between the inner and outer polymeric layers.

Example 38. The method of any example herein, particularly any one of Examples 34-37, wherein the steering member comprises a braided cable.

Example 39. The method of any example herein, particularly any one of Examples 34-37, wherein the steering member comprises a wire.

Example 40. A method for making a steerable catheter apparatus, the method comprising:
placing a first polymeric layer on a mandrel;
placing a steering member longitudinally along the first polymeric layer;
placing a braided layer over the first polymeric layer and the steering member;
positioning a pull ring relative to the first polymeric layer, the steering member and the braided layer such that a distal end portion of the braided layer extends into a proximal segment of the pull ring, a distal end portion of the steering member extends through the proximal segment of the pull ring, distally beyond the braided layer and alongside a distal segment of the pull ring, wherein proximal segment of the pull ring has a larger diameter than a diameter of the distal segment of the pull ring;
placing a second polymeric layer over the braided layer; and
bonding the first and second polymeric layers to each other via openings in the braided layer.

Example 41. The method of any example herein, particularly Example 40, wherein the steering member comprises a braided cable.

Example 42. The method of any example herein, particularly Example 41, wherein the steering member comprises a wire.

Example 43. The method of any example herein, particularly any one of Examples 40-42, wherein bonding the first and second polymeric layers to each other comprises applying heat and pressure to at least the second polymeric layer.

Example 44. The method of any example herein, particularly Example 43, wherein applying heat and pressure comprises placing a heart shrink layer over the second polymeric layer and heating the heat shrink layer.

Example 45. The method of any example herein, particularly any one of Examples 40-44, wherein the pull ring comprises a plurality of apertures and the first and second polymeric layers are bonded to each other via openings in the pull ring.

Example 46. The method of any example herein, particularly any one of Examples 40-45, wherein the pull ring further comprises a transition section formed between the proximal segment and the distal segment with an opening which is in communication with a groove formed on an outer surface of the distal segment of the pull ring, and wherein the distal end portion of the steering member extends through the proximal segment of the pull ring, the opening, and into the groove.

Example 47. The method of any example herein, particularly Example 46, wherein positioning the pull ring comprise aligning the groove of the pull ring with a longitudinally extending groove of the mandrel.

Example 48. The method of any example herein, particularly any one of Examples 40-47, wherein the steering member is inside of a tubular liner and placing the steering member comprises placing the tubular liner with the steering member inside the tubular liner longitudinally along the first polymer layer.

In view of the many possible embodiments to which the principles of the disclosure may be applied, it should be recognized that the illustrated embodiments are only preferred examples of the invention and should not be taken as limiting the scope of the disclosure. Rather, the scope of the disclosure is defined by the following claims.

The invention further comprises the following embodiments:
1. A delivery apparatus for an implantable medical device, comprising:
   a handle;
   a shaft extending distally from the handle, the shaft having a steering-member lumen extending a length of the shaft;
   a steering member extending through the steering-member lumen, the steering member having a proximal end portion and a distal end portion; and
   a pull ring disposed along a distal end portion of the shaft, the pull ring comprising a proximal segment having a first diameter and a distal segment having a second diameter smaller than the first diameter, wherein the distal end portion of the steering member is fixed to the distal segment of the pull ring;
   wherein the handle comprises an adjustment mechanism operatively coupled to the proximal end portion of the steering member and configured to adjust tension in the steering member so as to adjust a curvature of the shaft.
2. The delivery apparatus of embodiment 1, wherein the pull ring further comprises a transition section formed between the proximal segment and the distal segment with an opening which is in communication with a groove formed on an outer surface of the distal segment of the pull ring.
3. The delivery apparatus of embodiment 2, wherein the distal end portion of the steering member extends through the proximal segment of the pull ring, the opening, and into the groove.
4. The delivery apparatus of any previous embodiment, wherein the shaft comprises a braided layer radially outward of the steering-member lumen, wherein the braided layer has a distal end portion that extends into the proximal segment of the pull ring.
5. The delivery apparatus of embodiment 4, wherein the distal end portion of the steering member extends distally beyond a distal terminal end of the braided layer.
6. The delivery apparatus of any of embodiments 4 or 5, wherein the shaft comprises an inner polymeric layer defining the steering-member lumen and an outer polymeric layer, wherein the inner polymeric layer is radially inward of the braided layer and the outer polymeric layer is radially outward of the braided layer.
7. The delivery apparatus of embodiment 6, wherein the pull ring is embedded within the shaft between the inner and outer polymeric layers.
8. The delivery apparatus of any previous embodiment, wherein the steering member comprises a braided cable.
9. The delivery apparatus of any of embodiments 1 to 7, wherein the steering member comprises a wire.
10. The delivery apparatus of any previous embodiment, wherein a length of the proximal segment is shorter than a length of the distal segment.
11. The delivery apparatus of any previous embodiment, wherein a surface of one or both of the proximal and distal segments of the pull ring is perforated.
12. The delivery apparatus of any previous embodiment, wherein the shaft is an outer shaft having a central lumen, the delivery apparatus further comprising an inner shaft extending coaxially through the central lumen of the outer shaft, and wherein the steering-member lumen is radially offset from a central axis of the central lumen.
13. A steerable delivery apparatus for an implantable medical device, comprising:
   a handle;
   a shaft extending distally from the handle, the shaft having a steering-member lumen extending between a distal end portion and a proximal end portion of the shaft, and a braided layer radially outward of the steering-member lumen;
   a steering member extending through the steering-member lumen, the steering member having a proximal end portion and a distal end portion; and
   a pull ring coupled to the distal end portion of the shaft, the pull ring comprising a proximal segment having a first diameter and a distal segment having a second diameter smaller than the first diameter, wherein the distal end portion of the steering member is fixed to the distal segment of the pull ring, and wherein a distal end portion of the braided layer extends into the proximal segment of the pull ring and terminates at a location proximal to the distal segment of the pull ring;
   wherein the handle comprises an adjustment mechanism operatively coupled to the proximal end portion of the steering member, wherein actuation of the adjustment mechanism varies tension in the steering member to vary a curvature of the shaft.
14. The delivery apparatus of embodiment 13, wherein the pull ring further comprises a transition section formed between the proximal segment and the distal segment with an opening which is in communication with a groove formed on an outer surface of the distal segment of the pull ring, and wherein the distal end portion of the steering member extends through the proximal segment of the pull ring, the opening, and into the groove.
15. The delivery apparatus of embodiment 14, wherein the transition section tapers from the proximal segment to the distal segment of the pull ring.
16. The delivery apparatus of any of embodiments 13 to 15, wherein a distal terminal end of the steering member extends distally beyond a distal terminal end of the braided layer.
17. The delivery apparatus of any of embodiments 13 to 16, wherein the shaft comprises:
   an inner polymeric layer defining the steering-member lumen, the inner polymeric layer disposed radially inward of the braided layer; and
   an outer polymeric layer disposed radially outward of the braided layer, and wherein the pull ring is embedded within the distal end portion of the shaft between the inner and outer polymeric layers.
18. The delivery apparatus of any embodiments 13 to 17, wherein a surface of one or both of the distal and proximal segments of the pull ring comprises perforations.
19. The delivery apparatus of any embodiments 13 to 18, wherein the steering member comprises a braided cable.
20. The delivery apparatus of any of embodiments 13 to 18, wherein the steering member comprises a wire.
21. The delivery apparatus of any of embodiments 13 or 16 to 20, wherein the distal end portion of the steering member extends through the proximal segment of the pull ring, radially outwardly through an opening in the pull ring, and alongside an outer surface of the distal segment of the pull ring.
22. The delivery apparatus of embodiment 21, wherein the distal end portion of the steering member extends into a groove in the distal segment of the pull ring.
23. The delivery apparatus of any of embodiments 13 to 22, wherein the shaft is first shaft having a central lumen, the delivery apparatus further comprising a second shaft extending coaxially through the central lumen of the first shaft.
24. The delivery apparatus of embodiment 23, further comprising an inflatable balloon mounted on a distal end portion of the second shaft.
25. A method of using a catheter device, the method comprising:
   inserting a shaft of the catheter device into the body of a patient, the shaft comprising a steering-member lumen extending there-through, a steering member extending through the steering-member lumen, the steering member having a proximal end portion and a distal end portion, the distal end portion of the steering member fixed to a distal end portion of the shaft via a pull ring, the pull ring comprising a proximal segment having a first diameter and a distal segment having a second diameter, wherein the first diameter is larger than the second diameter; and
   steering the shaft inside the body of the patient by actuating an adjustment mechanism on a handle of the device outside the body of the patient, wherein the adjustment mechanism is operatively coupled to the proximal end portion of the steering member and wherein the actuating includes adjusting tension in the steering member to adjust a curvature of the shaft.
26. The method of embodiment 25, wherein the shaft comprises a braided layer radially outward of the steering-member lumen, wherein a distal end portion of the braided layer extends into the proximal segment.
27. A method for making a steerable catheter apparatus, the method comprising:
   placing a first polymeric layer on a mandrel;
   placing a steering member longitudinally along the first polymeric layer;
   placing a braided layer over the first polymeric layer and the steering member;
   positioning a pull ring relative to the first polymeric layer, the steering member and the braided layer such that a distal end portion of the braided layer extends into a proximal segment of the pull ring, a distal end portion of the steering member extends through the proximal segment of the pull ring, distally beyond the braided layer and alongside a distal segment of the pull ring, wherein proximal segment of the pull ring has a larger diameter than a diameter of the distal segment of the pull ring;
   placing a second polymeric layer over the braided layer; and
   bonding the first and second polymeric layers to each other via openings in the braided layer.
28. The method of embodiment 27, wherein the steering member comprises a braided cable.

## Claims

1. A pull ring (210) for a steerable delivery apparatus (100; 500) for an implantable medical device (10; 50), the pull ring (210) comprising a proximal segment (250) having a first diameter (252) and a distal segment (254) having a second diameter (256) smaller than the first diameter (252), wherein the distal segment (254) of the pull ring (210) is configured to be fixed to a distal end portion (226) of a steering member (202) of the steerable delivery apparatus (100; 500).

2. The pull ring (210) of claim 1, comprising a transition section (258) formed between the proximal segment (250) and the distal segment (254).

3. The pull ring (210) of claim 2, wherein the transition section (258) is formed with an opening (260).

4. The pull ring (210) of claim 3, wherein the opening (260) is provided on only one side of the transition section (258).

5. The pull ring (210) of claims 3 or 4, wherein the proximal segment (250) and the distal segment (254) are discontinuous from each other only at the location of the opening (260).

6. The pull ring (210) of at least one of claims 3 to 5, wherein the opening (260) is sized to be large enough to receive the steering member (202) therethrough.

7. The pull ring (210) of at least one of claims 3 to 5, wherein the opening (260) is in communication with a groove (262) formed on an outer surface of the distal segment (254) of the pull ring (210).

8. The pull ring (210) of claim 7, wherein the groove (262) extends along an entire length of the distal segment (254).

9. The pull ring (210) of at least one of the preceding claims, wherein the implantable medical device (10; 50) is a prosthetic heart valve.

10. The pull ring (210) of at least one of the preceding claims, wherein a length of the proximal segment (250) is shorter than a length of the distal segment (254).

11. The pull ring (210) of at least one of claims 1 to 9, wherein a length of the proximal segment (250) is longer than a length of the distal segment (254) or a length of the proximal and distal segments (250, 254) are equal or substantially equal.

12. The pull ring (210) of at least one of the preceding claims, wherein a surface of one or both of the proximal and distal segments (250, 254) of the pull ring (210) is perforated.

13. The pull ring (210) of at least one of the preceding claims, wherein the transition section (258) tapers from the proximal segment (250) to the distal segment (254) of the pull ring (210).

14. A steerable delivery apparatus (100; 500) for an implantable medical device (10; 50), the delivery apparatus (100; 500) comprising:
- a handle (102) comprising an adjustment mechanism (134),
- a shaft (200; 400) extending distally from the handle (102),
- the pull ring (210) of at least one of the preceding claims disposed on a distal portion of a the shaft (200; 400), and
- a steering member (202) that is affixed to the pull ring (210) at a distal end portion (226) and that is operatively connected to the adjustment mechanism at a proximal end portion, wherein
the adjustment mechanism (134) is operatively coupled to the proximal end portion of the steering member (202) and configured to adjust the tension of the steering member (202), thereby adjusting the curvature of the shaft (200; 400).

15. The steerable delivery apparatus (100; 500) of claim 14, wherein the pull ring (210) is embedded between an inner layer (214) and an outer layer (216) of the shaft (200; 400), which fixes the position of the pull ring (210) at a fixed location along the distal portion of the shaft (200; 400).
